# EUROPEAN PATENT APPLICATION

(11) **EP 4 684 648 A1**
(43) Date of publication of application: **28.01.2026**
(21) Application number: 24220255.4
(22) Date of filing: 16.12.2024
(51) Int. Cl.: A23J 1/00, A23J 1/10, A23J 3/04, A23J 3/30, A23K 10/26, A23K 20/147, B09B 3/45, C12P 21/06

(54) **METHOD FOR HYDROLYZING PROTEIN-RICH WASTE**

(30) Priority: 26.07.2024 TW 113127867
(71) Applicant: Riqian International Holdings Co., Ltd., Taoyuan City 335 (TW)
(72) Inventor: LEE, Ming-Chiu, 337 Taoyuan City (TW); SUN, Hao-Lun, 237 New Taipei City (TW)
(74) Representative: V.O.

(57) **Abstract**

A method for hydrolyzing protein-rich waste includes the steps of: A) pouring protein-rich waste and water into an outer shell (21) of a hydrolysis furnace device (2); B) drawing a portion of air out of the outer shell (21) using a negative pressure device (3); C) introducing inert gas into the outer shell (21) using an air cylinder device (4); D) introducing steam into the outer shell (21) using a steam boiler device(5); E) stirring the protein-rich waste and the water in the outer shell (21) using a stirring module (22) to generate a hydrolysis reaction; F) discharging the protein-rich waste to a separation device (6); G) performing a solid-liquid separation on the protein-rich waste using the separation device (6) to separate a protein-rich hydrolyzate semi-finished product; and H) reducing the moisture contained in the protein-rich hydrolyzate semi-finished product using a drying device (7) to produce a protein-rich hydrolyzate finished product.

## Description

The present disclosure relates to a hydrolysis method, more particularly to a method for hydrolyzing protein-rich waste.

In recent years, waste feather raw material was converted into feather powder or feather liquid that can be used as fertilizer or feed by hydrolysis technology, and this method has gradually become popular in the relevant fields. An existing hydrolysis method uses strong acid or base chemicals combined with low temperature and low pressure to hydrolyze the feather raw material so as to decompose keratin in the feather raw material into crude proteins.

However, the feather liquid decomposed by the existing hydrolysis method has a digestion rate ranging from 65% to 75%, so that the proteins in the waste feather raw material cannot be effectively used. Furthermore, the use of strong acid or base chemicals will generate related chemical wastes, incurring additional cleaning costs. Apparently, there is a need to conduct research and improvement of the existing hydrolysis method by those skilled in the relevant art.

Therefore, an object of the present disclosure is to provide a method for hydrolyzing protein-rich waste that can alleviate at least one of the drawbacks of the prior art.

According to this disclosure, the method for hydrolyzing protein-rich waste is implemented in conjunction with at least one hydrolysis furnace device, a negative pressure device, a air cylinder device for storing inert gas, a steam boiler device, a separation device and a drying device. The at least one hydrolysis furnace device includes an outer shell and a stirring module located inside the outer shell. The outer shell defines an air extraction opening for communicating with the negative pressure device, an air inlet for communicating with the air cylinder device, a steam port for communicating with the steam boiler device, an inlet opening, and an outlet opening. The method for hydrolyzing protein-rich waste comprises the steps of:
A) opening the inlet opening and simultaneously closing the air extraction opening, the air inlet, the steam port, and the outlet opening, followed by pouring protein-rich waste and water into the outer shell through the inlet opening, after which the inlet opening is closed;
B) opening the air extraction opening, followed by drawing a portion of air out of the outer shell using the negative pressure device through the air extraction opening, after which the air extraction opening is closed;
C) opening the air inlet, followed by introducing the inert gas into the outer shell using the air cylinder device through the air inlet, after which the air inlet is closed;
D) opening the steam port, followed by introducing steam into the outer shell using the steam boiler device through the steam port, after which the steam port is closed;
E) stirring the protein-rich waste and the water in the outer shell using the stirring module for a reaction time length to generate a hydrolysis reaction;
F) opening the outlet opening to discharge the protein-rich waste to the separation device after the hydrolysis reaction;
G) performing a solid-liquid separation on the protein-rich waste using the separation device after the hydrolysis reaction to separate a protein-rich hydrolyzate semi-finished product, and discharging the protein-rich hydrolyzate semi-finished product to the drying device; and
H) reducing the moisture contained in the protein-rich hydrolyzate semi-finished product using the drying device to produce a protein-rich hydrolyzate finished product.

Other features and advantages of the disclosure will become apparent in the following detailed description of the embodiment with reference to the accompanying drawings. It is noted that various features may not be drawn to scale.
FIG. 1 is a block diagram of a protein-rich waste hydrolysis system used in conjunction with an embodiment of a method for hydrolyzing protein-rich waste according to the present disclosure.
FIG. 2 is a perspective view of one of the hydrolysis furnace devices of the protein-rich waste hydrolysis system.
FIG. 3 is a schematic sectional view of FIG. 2.
FIG. 4 is a flow chart, illustrating the steps involved in the method according to the embodiment of this disclosure.
FIG. 5 is another flow chart, illustrating sub-steps of step S6 of the method of FIG. 4.

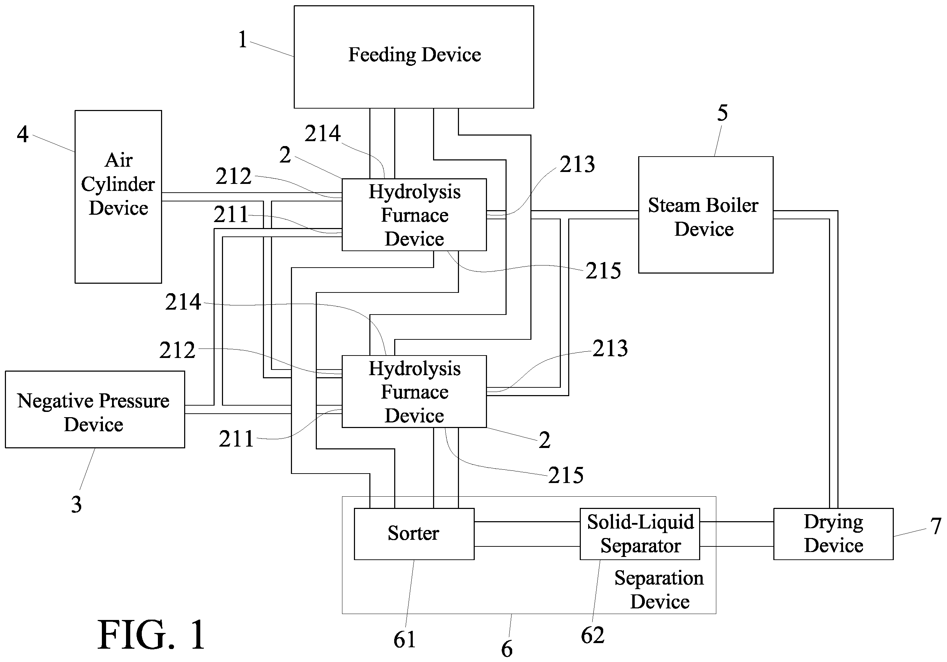

Referring to FIG. 1, a method for hydrolyzing protein-rich waste according to an embodiment of the present disclosure is implemented in conjunction with a protein-rich waste hydrolysis system to generate a hydrolysis reaction of protein-rich waste (not shown). The protein-rich waste hydrolysis system includes a feeding device 1, two hydrolysis furnace devices 2, a negative pressure device 3, an air cylinder device 4 for storing inert gas, a steam boiler device 5, a separation device 6 and a drying device 7. It is worth mentioning that the number of the hydrolysis furnace device 2 may be adjusted to one or more than two according to the amount of the protein-rich waste, and is not limited thereto. In this embodiment, the protein-rich waste is composed of at least one of livestock feathers and livestock scraps (e.g., the head portions and the claw portions).

The feeding device 1 is used for distributing the protein-rich waste to the hydrolysis furnace devices 2 at a fixed speed, enabling the protein-rich waste to be quantitatively distributed to the hydrolysis furnace devices 2. In this embodiment, the feeding device 1 is implemented through cooperation of a screw conveyor and a hopper having an upper cover (not shown).

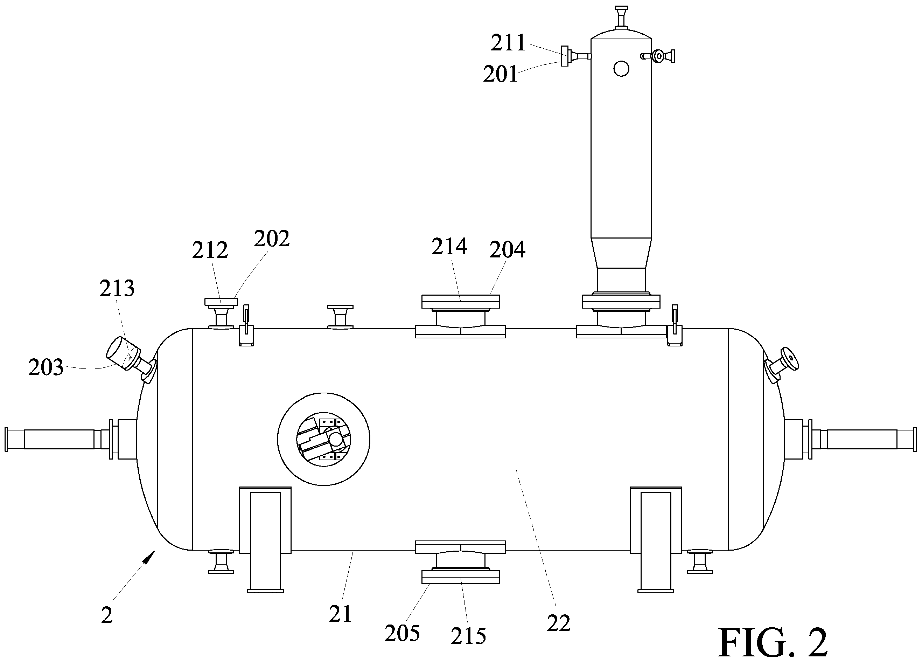

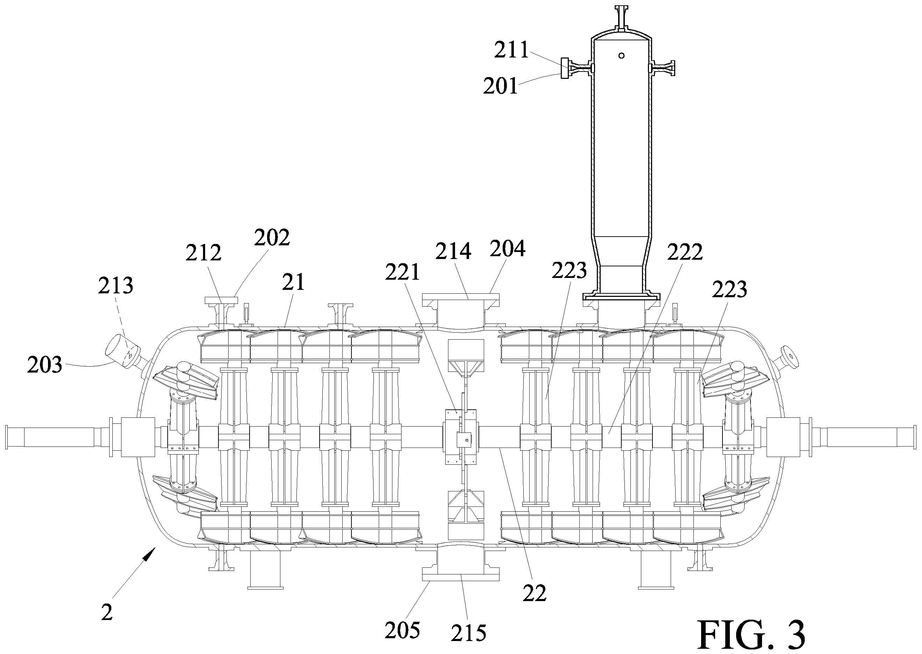

Referring to FIGS. 2 and 3, each hydrolysis furnace device 2 includes an outer shell 21, an air extraction valve 201, an air inlet valve 202, a steam valve 203, a feed valve 204 and a discharge valve 205 all disposed on the outer shell 21, a stirring module 22 located inside the outer shell 21, and a control module (not shown) electrically connected to the air extraction valve 201, the air inlet valve 202, the steam valve 203, the feed valve 204, the discharge valve 205 and the stirring module 22. The outer shell 21 defines an air extraction opening 211 for communicating with the negative pressure device 3, an air inlet 212 for communicating with the air cylinder device 4, a steam port 213 for communicating with the steam boiler device 5, an inlet opening 214, and an outlet opening 215. The air extraction valve 201 is disposed on and connected to the air extraction opening 211. The air inlet valve 202 is disposed on and connected to the air inlet 212. The steam valve 203 is disposed on and connected to the steam port 213. The feed valve 204 is disposed on and connected to the inlet opening 214. The discharge valve 205 is disposed on and connected to the outlet opening 215. The stirring module 22 includes a carrier rod 222 rotatably disposed in the outer shell 21, and a plurality of spaced-apart stirring rods 223 fixed to the carrier rod 222.

In this embodiment, the air extraction valves 201, the air inlet valves 202, the steam valves 203, the feed valves 204, and the discharge valves 205 of the hydrolysis furnace devices 2 are all ball valves. The control module can be implemented by hardware or firmware, such as field-programmable gate array (FPGA), microprocessor, or system on chip. Since the control module is common knowledge in the field of automatic control, and is not an important feature of this disclosure, a detailed description thereof will be omitted herein.

Referring back to FIGS. 1 and 3, the negative pressure device 3 is used for performing a vacuum extraction operation on the hydrolysis furnace devices 2 through the air extraction openings 211 thereof. In this embodiment, the negative pressure device 3 is a vacuum pump or an induced draft fan.

The air cylinder device 4 is used for supplying and replenishing inert gas into the outer shells 21 of the hydrolysis furnace devices 2 through the air inlets 212 thereof. In this embodiment, the air cylinder device 4 is a high-pressure steel cylinder.

The steam boiler device 5 is used for heating water to steam and then introducing the steam into the outer shells 21 through the steam ports 213 thereof, so that the pressure in each outer shell 21 ranges from 14 to 22 kgf/cm2.

The separation device 6 includes a sorter 61 and a solid-liquid separator 62. The sorter 61 is used for sorting the protein-rich waste after hydrolysis reaction according to particle size to separate a first sorted material and a second sorted material. When the particle size of the second sorted material is greater than that of the first sorted material, the second sorted material must be collected and sent back to the hydrolysis furnace devices 2 to continue the hydrolysis reaction. The solid-liquid separator 62 is used for performing solid-liquid separation on the first sorted material to separate a protein-rich hydrolyzate semi-finished product.

The drying device 7 is used for reducing moisture contained in the protein-rich hydrolyzate semi-finished product to produce a protein-rich hydrolyzate finished product.

To briefly introduce the operation method of this embodiment, only one of the hydrolysis furnace devices 2 will be taken as an example for description below.

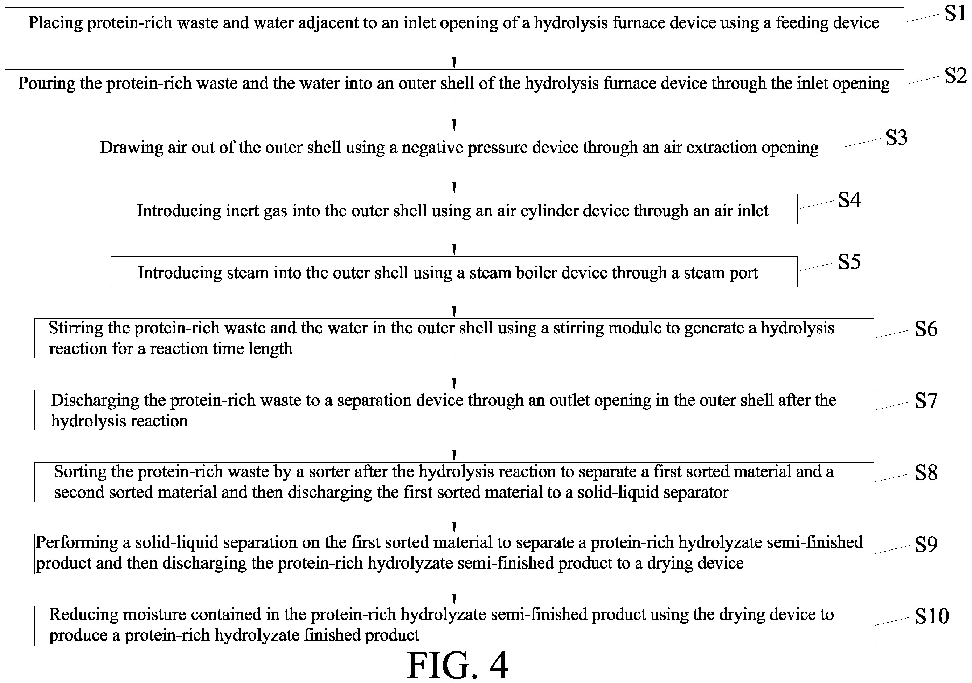

Specifically, referring to FIGS. 1 to 4, the method for hydrolyzing the protein-rich waste of this embodiment includes steps S1 to S10.

In step S1, protein-rich waste and water are placed adjacent to the inlet opening 214 of the outer shell 21 of the hydrolysis furnace device 2 using the feeding device 1 .

In step S2, the feed valve 204 is actuated to open the inlet opening 214, the air extraction valve 201 is actuated to close the air extraction opening 211, the air inlet valve 202 is actuated to close the air inlet 212, the steam valve 203 is actuated to close the steam port 213, the discharge valve 205 is actuated to close the outlet opening 215, and the protein-rich waste and the water are poured into the outer shell 21 through the inlet opening 214, after which the feed valve 204 is again actuated to close the inlet opening 214.

In step S3, the air extraction valve 201 is actuated to open the air extraction opening 211, and a portion of air in the outer shell 21 is drawn out using the negative pressure device 3 through the air extraction opening 211, after which the air extraction valve 201 is again actuated to close the air extraction opening 211.

In step S4, the air inlet valve 202 is actuated to open the air inlet 212, and the inert gas is introduced into the outer shell 21 using the air cylinder device 4 through the air inlet 212, after which the air inlet valve 202 is again actuated to close the air inlet 212. In this step, by introducing the inert gas into the outer shell 21, a peroxidation reaction between the oxygen-containing gas and the protein-rich waste can be reduced during the hydrolysis reaction, thereby preventing poor color quality and quality degradation of the protein-rich hydrolyzate finished product.

In step S5, the steam valve 203 is actuated to open the steam port 213, and steam is introduced into the outer shell 21 using the steam boiler device 5 through the steam port 213, after which the steam valve 203 is again actuated to close the steam port 213. At this time, the pressure in the outer shell 21 ranges from 14 to 22 kgf/cm2.

It is worth mentioning that, in a variation of this embodiment, when the outer shell 21 of the hydrolysis furnace device 2 receives the protein-rich waste and the water through the inlet opening 214, the stirring module 22 can be actuated to start stirring the protein-rich waste and the water for an auxiliary reaction time length. The auxiliary reaction time length depends on the time spent in steps S2 to S5.

In step S6, the protein-rich waste and the water in the outer shell 21 are stirred by the stirring module 22 to generate a hydrolysis reaction for a reaction time length. In other words, the process of stirring the protein-rich waste and the water by the stirring module 22 will correspond to a sum of the auxiliary reaction time length and the reaction time length.

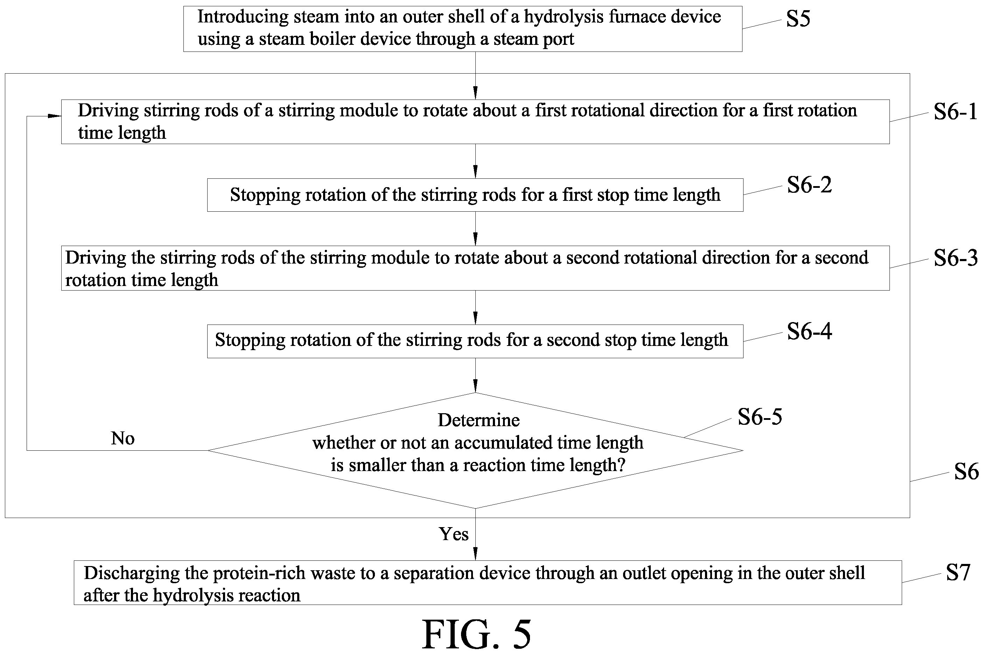

Referring to FIG. 5, in combination with FIG. 3, step S6 includes sub-steps S6-1 to S6-5.

In sub-step S6-1, the stirring rods 223 of the stirring module 22 are driven to rotate about a first rotational direction (forward rotation) for a first rotation time length.

In sub-step S6-2, the rotation of the stirring rods 223 is stopped for a first stop time length.

In sub-step S6-3, the stirring rods 223 are driven to rotate about a second rotational direction (reverse direction) opposite to the first rotational direction for a second rotation time length.

In sub-step S6-4, the rotation of the stirring rods 223 is stopped for a second stop time length.

In sub-step S6-5, a sum of the first rotation time length, the first stop time length, the second rotation time length, and the second stop time length is defined as an accumulated time length. If the accumulated time length is smaller than the reaction time length, sub-steps S6-1 to S6-4 are repeated. If the accumulated time length is not less than the reaction time length, then proceed to step S7.

It is worth mentioning that each of the first and second rotation time lengths is preset at 5 minutes, each of the first and second stop time lengths is preset at 15 seconds, and the reaction time length ranges from 30 to 60 minutes. In other words, it takes 10 minutes and 30 seconds to perform sub-steps S6-1 to S6-4, and thus, it will take longer than the reaction time length to repeat sub-steps S6-1 to S6-4 for three to six times. Step S7 is performed afterwards.

In step S7, referring back to FIGS. 1 to 4, the discharge valve 205 is actuated to open the outlet opening 215, and the protein-rich waste is discharged to the separation device 6 after the hydrolysis reaction.

In step S8, the protein-rich waste after the hydrolysis reaction is sorted by the sorter 61 of the separation device 6 to separate a first sorted material and a second sorted material, and the first sorted material is discharged to the solid-liquid separator 62.

In step S9, the solid-liquid separator 62 of the separation device 6 is used to perform a solid-liquid separation on the first sorted material so as to separate the protein-rich hydrolyzate semi-finished product, and the protein-rich hydrolyzate semi-finished product is discharged to the drying device 7.

In step S10, the drying device 7 is used to reduce moisture contained in the protein-rich hydrolyzate semi-finished product so as to produce the protein-rich hydrolyzate finished product.

In summary, by performing the steps of the method of this embodiment, the inert gas and the heated steam are introduced sequentially into the outer shell 21, thereby providing high pressure and high temperature method to hydrolyze the protein-rich waste and reducing the peroxidation reaction between the oxygen-containing gas contained in the outer shell 21 and the protein-rich waste. Thus, poor color quality and quality degradation of the protein-rich hydrolyzate finished product can be prevented, and related chemical wastes will not be discharged, so the method of this disclosure can simultaneously improve the quality of the protein-rich hydrolyzate finished product and reduce the chemical wastes.

Furthermore, with the stirring rods 223 being driven to rotate about the first rotational direction (forward direction), then stop, then rotate about the second rotational direction (reverse direction), and then stop again, this will determine whether to continue or not the ongoing stirring process, so that not only can the uniformity of the protein-rich waste be enhanced by changing the rotational direction, but the protein-rich waste can also be allowed to layered naturally when stopped to perform the hydrolysis reaction. Moreover, the hydrolysis furnace device 2 can be prevented from overheating or generating mechanical fatigue due to a long period of operation. Therefore, the quality of the protein-rich hydrolyzate finished product can be improved, and the service life of the hydrolysis furnace device 2 can be prolonged.

In addition, the protein-rich hydrolyzate finished product can be made into feeds, fertilizers, artificial joints, etc., and has quite a wide range of applications.

Therefore, the method for hydrolyzing protein-rich waste of this disclosure has a good hydrolysis effect, and can indeed achieve the object thereof.

In the description above, for the purposes of explanation, numerous specific details have been set forth in order to provide a thorough understanding of the embodiments. It will be apparent, however, to one skilled in the art, that one or more other embodiments may be practiced without some of these specific details. It should also be appreciated that reference throughout this specification to "one embodiment," "an embodiment," an embodiment with an indication of an ordinal number and so forth means that a particular feature, structure, or characteristic may be included in the practice of the disclosure. It should be further appreciated that in the description, various features are sometimes grouped together in a single embodiment, figure, or description thereof for the purpose of streamlining the disclosure and aiding in the understanding of various inventive aspects; such does not mean that every one of these features needs to be practiced with the presence of all the other features. In other words, in any described embodiment, when implementation of one or more features or specific details does not affect implementation of another one or more features or specific details, said one or more features may be singled out and practiced alone without said another one or more features or specific details. It should be further noted that one or more features or specific details from one embodiment may be practiced together with one or more features or specific details from another embodiment, where appropriate, in the practice of the disclosure.

## Claims

1. A method for hydrolyzing protein-rich waste and implemented in conjunction with at least one hydrolysis furnace device (2), a negative pressure device (3), an air cylinder device (4) for storing inert gas, a steam boiler device (5), a separation device (6) and a drying device (7), the at least one hydrolysis furnace device (2) including an outer shell (21) and a stirring module (22) located inside the outer shell (21), the outer shell (21) defining an air extraction opening (211) for communicating with the negative pressure device (3), an air inlet (212) for communicating with the air cylinder device (4), a steam port (213) for communicating with the steam boiler device (5), an inlet opening (214), and an outlet opening (215), the method for hydrolyzing protein-rich waste comprising the steps of:
A) opening the inlet opening (214) and simultaneously closing the air extraction opening (211), the air inlet (212), the steam port (213), and the outlet opening (215), followed by pouring protein-rich waste and water into the outer shell (21) through the inlet opening (214), after which the inlet opening (214) is closed;
B) opening the air extraction opening (211), followed by drawing a portion of air out of the outer shell (21) using the negative pressure device (3) through the air extraction opening (211), after which the air extraction opening (211) is closed;
C) opening the air inlet (212), followed by introducing the inert gas into the outer shell (21) using the air cylinder device (4) through the air inlet (212), after which the air inlet (212) is closed;
D) opening the steam port (213), followed by introducing steam into the outer shell (21) using the steam boiler device (5) through the steam port (213), after which the steam port (213) is closed;
E) stirring the protein-rich waste and the water in the outer shell (21) using the stirring module (22) for a reaction time length to generate a hydrolysis reaction;
F) opening the outlet opening (215) to discharge the protein-rich waste to the separation device (6) after the hydrolysis reaction;
G) performing a solid-liquid separation on the protein-rich waste using the separation device (6) after the hydrolysis reaction to separate a protein-rich hydrolyzate semi-finished product, and discharging the protein-rich hydrolyzate semi-finished product to the drying device (7); and
H) reducing the moisture contained in the protein-rich hydrolyzate semi-finished product using the drying device (7) to produce a protein-rich hydrolyzate finished product.

2. The method for hydrolyzing protein-rich waste as claimed in claim 1, wherein the protein-rich waste is cooperatively hydrolyzed by a feeding device (1), the at least one hydrolysis furnace device (2), the negative pressure device (3), the air cylinder device (4), the steam boiler device (5), the separation device (6) and the drying device (7), the feeding device (1) communicating with the at least one hydrolysis furnace device (2), and wherein the method further comprises step I) prior to step A), in which the protein-rich waste and the water are placed adjacent to the inlet opening (214) using the feeding device (1).

3. The method for hydrolyzing protein-rich waste as claimed in claim 1, wherein the separation device (6) includes a sorter (61) and a solid-liquid separator (62), and wherein step G) includes:
G-1) sorting the protein-rich waste using the sorter (61) after the hydrolysis reaction to separate a first sorted material and a second sorted material, and discharging the first sorted material to the solid-liquid separator (62); and
G-2) performing a solid-liquid separation on the first sorted material using the solid-liquid separator (62) to separate the protein-rich hydrolyzate semi-finished product, and discharging the protein-rich hydrolyzate semi-finished product to the drying device (7).

4. The method for hydrolyzing protein-rich waste as claimed in claim 1, wherein the stirring module (22) includes a carrier rod (222) rotatably disposed in the outer shell (21), and a plurality of spaced-apart stirring rods (223) fixed to the carrier rod (222), and wherein step E) includes:
E-1) driving the stirring rods (223) to rotate about a first rotational direction for a first rotation time length;
E-2) stopping the rotation of the stirring rods (223) for a first stop time length;
E-3) driving the stirring rods (223) to rotate about a second rotational direction opposite to the first rotational direction for a second rotation time length;
E-4) stopping the rotation of the stirring rods (223) for a second stop time length; and
E-5) defining a sum of the first rotation time length, the first stop time length, the second rotation time length, and the second stop time length as an accumulated time length, wherein, if the accumulated time length is less than the reaction time length, steps E-1) to E-4) are repeated; and if the accumulated time length is not less than the reaction time length, then proceed to step F).

5. The method for hydrolyzing protein-rich waste as claimed in claim 1, wherein, in step A), the protein-rich waste and the water in the outer shell (21) are stirred using the stirring module (22) for an auxiliary reaction time length.

6. The method for hydrolyzing protein-rich waste as claimed in claim 1, wherein, in step A), the protein-rich waste is composed of at least one of livestock feathers and livestock scraps.
